Europäisches Patentamt

European Patent Office

Office européen des brevets

(11) Veröffentlichungsnummer: **0 042 499**
**B1**

(12) **EUROPÄISCHE PATENTSCHRIFT**

(45) Veröffentlichungstag der Patentschrift:
18.07.84

(51) Int. Cl.³: **A 61 G 9/00**, A 61 B 5/00

(21) Anmeldenummer: 81104063.3

(22) Anmeldetag: 27.05.81

(54) Flexibel ausgebildeter, einteiliger Auffangbehälter für medizinische Zwecke.

(30) Priorität: 21.06.80 DE 3023348

(43) Veröffentlichungstag der Anmeldung:
30.12.81 Patentblatt 81/52

(45) Bekanntmachung des Hinweises auf die Patenterteilung:
18.07.84 Patentblatt 84/29

(84) Benannte Vertragsstaaten:
AT CH FR GB LI NL SE

(56) Entgegenhaltungen:
FR - A - 2 178 022
FR - A - 2 426 619
US - A - 2 936 757
US - A - 4 095 589
US - A - 4 188 989

(73) Patentinhaber: Sterimed Gesellschaft für medizinischen Bedarf mbH, Fasanerieweg 15, D-6600 Saarbrücken (DE)

(72) Erfinder: Härle, Anton, Dr., Schelmenstiege 8, D-4400 Münster-Roxel (DE)

(74) Vertreter: Suchy, Herbert, Byk Gulden Lomberg Chemische Fabrik GmbH Patentabteilung Byk-Gulden-Strasse 2, D-7750 Konstanz (DE)

## Beschreibung

Die Erfindung bezieht sich auf einen flexibel ausgebildeten, einteiligen Auffangbehälter für medizinische Zwecke mit einem Meßbeutelteil, einen Hauptbeutelteil und einer Aufhängeeinrichtung, bei dem der mit einem Einlaufröhrchen ausgerüstete, eine Meßskaleneinteilung aufweisende und sich im Querschnitt verjüngende Meßbeutelteil dem Hauptbeutelteil in Strömungsrichtung vorgeschaltet ist und am Auslaßende unter Freihalten eines Durchganges einteilig in den Hauptbeutelteil übergeht.

Eine Vorrichtung der vorstehend definierten Art wird in der FR-A-2 178 022 beschrieben. Der Meßbeutelteil verjüngt sich bei der bekannten Vorrichtung von seinem Einlaßende zum Auslaßende hin und geht mit seinem Auslaßende einteilig in den Hauptbeutelteil über, wobei im Meßfall dieser Übergang durch eine medizinische Klemme verschlossen werden kann. Außerdem ist ein Überlaufkanal vorgesehen, der vom Meßbeutelteil in den Hauptbeutelteil führt. Der Einsatz einer medizinischen Klemme führt zu ungenauen Meßwerten, da Verformungen des Beutels auftreten und der Bereich, wie hoch die Klemme den eigentlichen Meßbeutelteil verschließt, nicht genau bestimmbar ist.

Exakte Messungen kleiner Flüssigkeitsmengen sind somit nur schwer möglich. Die Herstellung eines solchen Beutels mit den erforderlichen, vielen innerhalb des Beutels verlaufenden Schweißnähten ist konstenaufwendig. Die Strömungsmenge der in den Meßbeutelteil einfließenden Flüssigkeit, z. B. Urin, ist nicht kontrollierbar, da der durch das Katheterröhrchen ankommende Urin an den Beutelwandungen entlangläuft und nicht frei in den Meßbeutelteil hineintropfen kann.

Aus der US-A-4 095 589 ist ein Urinsammelbeutel bekannt, der aus flexiblem Material besteht und dem ein aus halbstarrem Werkstoff bestehendes Urometer vorgeschaltet ist. Dieses Urometer muß in einem gesonderten Arbeitsgang über eine nut-federartige Verbindung mit dem Hauptbeutelteil verbunden werden, so daß ein in der Herstellung aufwendiges Aggregat geschaffen wird, das nicht als Wegwerfbeutel geeignet ist. Aus diesem Grunde ist sowohl das Urometer wie auch der Hauptbeutelteil mit entsprechenden Entleerungsöffnungen ausgerüstet, so daß kein geschlossenes, sondern ein sogenanntes offenes System vorliegt, d. h. ein Zwischenspeicher, der nicht infektionssicher ist.

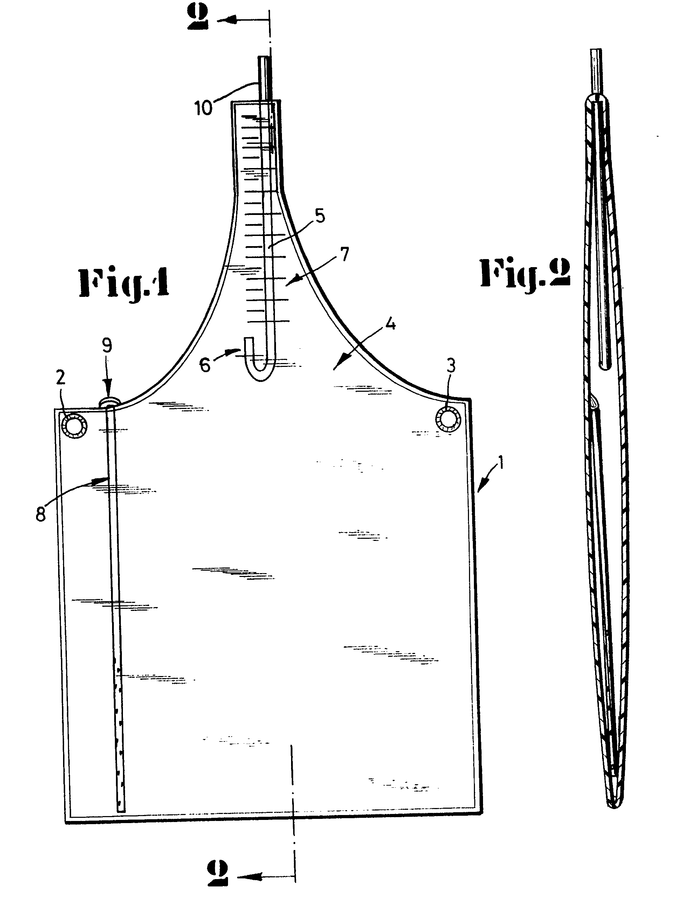

In der US-A-4 188 989 wird ein flexibler Beutel beschrieben, der als Probeentnahmevorrichtung gestaltet ist. Aufgabe dieses Beutels ist es, bei gefülltem Beutel kleine, vorbestimmte Probemengen entnehmen zu können. Der Beutel kann nicht als Urinmeßbeutel eingesetzt werden, denn in diesem Fall wäre es erforderlich, daß die Zulaufleitung von oben zum Meßteil führt. Der normale Einsatzfall ist die in Fig. 1 dargestellte Anordnung, und wenn eine Probe entnommen werden soll, wird der Beutel in die in Fig. 2 dargestellte Stellung geklappt.

Neben den rein technischen Nachteilen sowie der schlechten und aufwendigen Kontrolle des Urinflusses hatten die bekannten Systeme zudem den Nachteil, daß das Pflegepersonal und damit auch der Patient mit Keimen infiziert werden konnte. Die Erfahrungswerte bei der Patienteninfektion gehen dahin, daß sich bei dem bekannten Ableitsystem etwa 80% aller Patienten nach zwei Tagen infiziert haben, was zusätzliche Behandlungen notwendig macht, die natürlich vermieden werden sollten.

In der DE-A-2 438 154 wird eine Einrichtung beschrieben, die aus einem starren Kopfteil und einem sich daran anschließenden, fest verbundenen flexiblen Beutelteil besteht. Das starre Kopfteil besteht dabei aus einem formstabilen, vorzugsweise aus einem Duroplasten gefertigten Gerät, das mit Aufhängeeinrichtungen ausgerüstet ist. In diesem Kopfteil ist dann der Meßteil, d. h. die Kammer ausgeformt, die den eigentlichen Meßteil bildet. An die Kammer schließt sich eine Verbindungsleitung zum flexiblen Beutelteil an, wobei diese Verbindungsleitung von Hand betätigt werden muß. Weiterhin schließt sich an das flexible Beutelteil eine Entleerungsvorrichtung an, und in dem eigentlichen Kopfteil ist eine Luftöffnung vorgesehen, die mit einem Filter ausgerüstet sein kann und die über eine Leitung mit dem Inneren des flexiblen Beutelteiles in Verbindung steht.

Das bekannte Gerät ist außerordentlich sperrig, kostenaufwendig und stellt aufgrund des Luftabzuges kein vollständig geschlossenes Harnsammel- und -meßsystem dar.

Der Erfindung liegt die Aufgabe zugrunde, ein geschlossenes Flüssigkeitssammel- und meßsystem zu schaffen, bei welchem eine schnelle problemlose Kontrolle des jeweiligen Flusses möglich ist und bei welchem eine infektionssichere Speicherung der Flüssigkeit angestrebt wird, wobei der Beutel so kostengünstig hergestellt werden soll, daß er als Wegwerfbeutel ausgebildet sein kann.

Diese der Erfindung zugrunde liegende Aufgabe wird dadurch gelöst, daß der Meßbeutelteil sich zum Einlaßende hin verjüngt und in die Gebrauchslage um eine im Bereich der Aufhängeeinrichtung liegende Knickkante umklappbar ist, daß der Nullwert der Meßskaleneinteilung am äußersten Meßbeutelteilende beginnt und daß das Einlaufröhrchen in seinem freien Ende innerhalb des Meßbeutelteiles abgebogen ist.

Durch diese Maßnahme wird ein Auffangbehälter geschaffen, der mit dem gleichen Aufwand bezüglich der Herstellung wie die bisher bekannten einfach ausgebildeten Beutel hergestellt werden kann, wobei aber der erfindungsgemäße Beutel den Vorteil hat, daß nunmehr der Meßbeutelteil umgeklappt werden kann, und zwar um eine Knicklinie, die in der Ebene der Aufhängeeinrichtungen liegt, so daß theoretisch zwei Beutelteile geschaffen werden, nämlich einmal ein Meßbeutelteil, wobei dieser Meßteil durch den sich verjüngenden Beutelteil gebildet wird, und ein Hauptbeutelteil, in dem die größeren Flüssigkeitsmengen gespeichert werden, wobei dieser Hauptbeutelteil durch den sich an den verjüngenden Beutelteil anschließenden Beutelteil gebildet wird.

Der Überfluß von einem zum anderen Beutelteil erfolgt im Gegensatz zu der bekannten Einrichtung automatisch, und durch einfaches Anheben des umgebogenen Beutelteiles kann der Meßbeutelteil leergemacht werden, d. h. umgefüllt werden in den Hauptbeutelteil, und nunmehr ist sofort die Kontrolle des neu einfließenden Urins möglich.

Wird das Einlaufröhrchen mit einer Tropfnase ausgerüstet, die innerhalb des Rohrraumes liegt, erfolgt das Einlaufen der Flüssigkeit in den Meßbeutelteil nun nicht am Rande des Beutels, sondern z. B. der Urin muß von dem Einlaufröhrchen heruntertropfen, so daß dadurch sofort sichtbar ist, in welcher Form mit welcher Menge der Urin vom Patienten abgegeben wird.

Es ist, ohne mit der Flüssigkeit in Verbindung zu kommen, ein leichtes Entleeren des sich konisch verjüngenden Meßbeutelteiles in den Hauptbeutelteil möglich, so daß jederzeit wieder neu gemessen werden kann, wobei auch der Hauptbeutelteil mit einer Skaleneinteilung ausgerüstet sein kann, so daß die insgesamt abgegebene Urinmenge dort gemessen werden kann.

Ein Entleeren des Behälters kann einmal in an sich bekannter Weise durch Abnahme des Behälters vom Katheter erfolgen, es ist aber auch gemäß einem besonderen Vorschlag der Erfindung möglich, das Entleeren des Behälters durch die Durchstechkappe und das Entleerungsröhrchen hindurch vorzunehmen, wobei dann ein Kontakt des Pflegers mit der Flüssigkeit des Patienten überhaupt von Keimen weitgehend ausgeschlossen wird.

Der erfindungsgemäße Auffangbehälter, der kostengünstig im laufender Produktion aus Plastikfolien hergestellt werden kann, löst die Vielzahl der vorstehend angeschnittenen Probleme in denkbar einfachster Weise und gibt dem Praktiker ein Hilfsmittel an die Hand, das ihm die tägliche Arbeit wesentlich erleichtert und für das Pflegepersonal verbesserte Arbeitsbedingungen schafft. Insbesondere wird ein sogenanntes geschlossenes System geschaffen, bei dem eine Unterbrechung beispielsweise zu Entleerungszwecken oder zu Meßzwecken nicht mehr notwendig ist. Neben der einfachen Bedienungsmöglichkeit wird die Entsorgung vereinfacht und stets mit dem Behälter ein Urometer mitgeliefert, ohne daß dazu besondere Kosten entstehen.

Ein Ausführungsbeispiel der Erfindung als Urinbeutel wird nachfolgend anhand der Zeichnungen erläutert. Die Zeichnungen zeigen dabei in

Fig. 1 eine Darstellung des Urinbeutels bei hochgeklapptem Meßbeutelteil, in

Fig. 2 einen Schnitt gemäß der Linie 2-2 in Fig. 1, in

Fig. 3 den Urinbeutel in der Gebrauchslage mit abgeklapptem Meßbeutelteil und in

Fig. 4 einen Schnitt gemäß der Linie 4-4 in Fig. 3 sowie in

Fig. 5 in einem wesentlich größeren Maßstab die Anordnung einer Tropfnase innerhalb des Rohrendes des Einlaufröhrchens.

In den Zeichnungen ist ein Hauptbeutelteil 1 dargestellt, der mit einer Aufhängeeinrichtung bestehend aus den Ösen 2 und 3 ausgerüstet ist. An die im Bereich der Ösen 2 und 3 verlaufende Knickkante schließt sich an den Hauptbeutelteil 1 ein sich konisch verjüngender Meßbeutelteil 4 an, in dessen oberem Teil ein Einlaufröhrchen 5 eingearbeitet ist, das dicht mit der Oberkante des Meßbeutelteiles 4 verschweißt wird. Das Einlaufröhrchen 5 ist an seinem freien, im Beutel befindlichen Ende halbkreisförmig, wie dies bei 6 dargestellt ist, abgebogen. Außerdem weist der Meßbeutelteil eine Meßskaleneinteilung 7 auf.

Der Meßbeutelteil 4 ist etwas versetzt angeordnet, so daß bei der Darstellung gemäß Fig. 1 im linken Teil der Figur ein Raum zur Aufnahme eines Entnahmeröhrchens 8 geschaffen wird, wobei dieses Entnahmeröhrchen 8 am unteren Ende perforiert ist und mit einer Durchstechkappe 9 am oberen Ende des Hauptbeutelteiles 1 mündet. Die Durchstechkappe 9 besteht, wie dies an sich bekannt ist, aus einem elastischen Werkstoff, vorzugsweise Gummi, Kunstkautschuk od. dergl. und weist eine Stärke auf, die auch bei mehrmaliger Funktion mittels einer Kanüle eine gute Dichtigkeit beibehält, wobei diese Dichtigkeit sowohl die Abdichtung des durchdrungenen Bereiches gegenüber der Kanüle als auch das dichte Verschließen der Funktionsöffnung nach dem Zurückziehen der Kanüle gewährleisten muß.

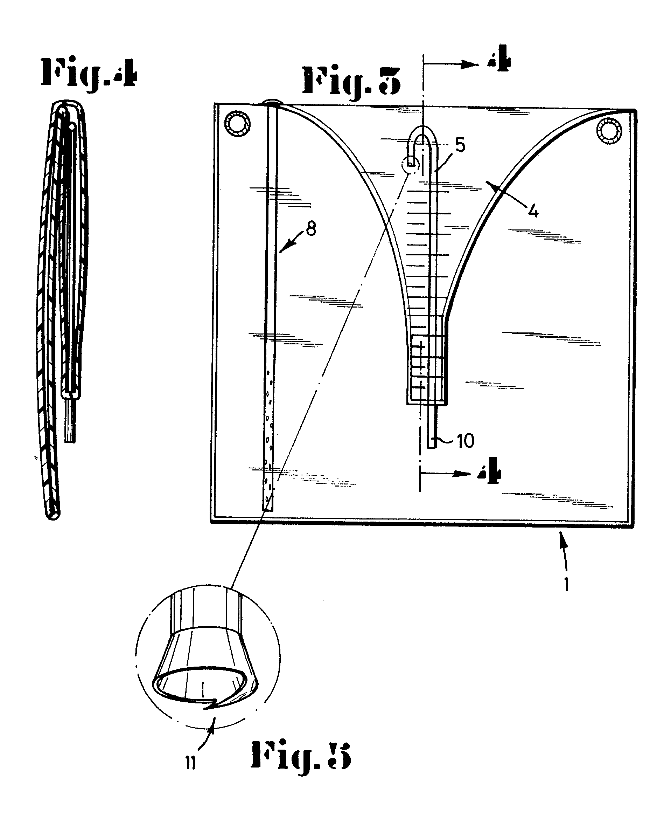

Aus der Darstellung von Fig. 3 ist ersichtlich, daß in der Gebrauchslage der Meßbeutelteil 4 um die im Bereich der Aufhängeeinrichtung 2, 3 liegende Knickkante umgebogen werden kann, so daß nunmehr die Zuführung des Urins durch den Anschlußteil 10 des Einlaufröhrchen 5 erfolgt. Der Urin tropft dabei aus dem umgebogenen Teil 6 in den sich nach unten hin nunmehr konisch verjüngenden Meßbeutelteil 4, und hier kann an der vorhandenen Meßskaleneinteilung 7 die Urinmenge abgelesen werden.

Außerdem ist der Hauptbeutelteil 1 mit einer Meßskaleneinteilung ausgerüstet, die das Messen der aus dem Meßbeutelteil 4 in den Hauptbeutelteil 1 abgegebenen Urinmenge ermöglicht.

Es ist ersichtlich, daß bei Benutzung des Auffangbehälters einerseits ein automatisches Überfließen des Urins aus dem Meßbeutelteil 4 in den Hauptbeutelteil 1 erfolgt, daß aber auch jederzeit ein Entleeren des Meßbeutelteiles 4 in den Hauptbeutelteil 1 möglich ist, so daß stets zu jeder Zeit von neuem die Urinmessung in der zur Verfügung stehenden Zeiteinheit gemessen werden kann.

Fig. 5 zeigt, daß sich das umgebogene Ende 6 des Einlaufröhrchens 5 konisch erweitert und mit einer Tropfnase 11 ausgerüstet ist, die sicherstellt, daß kein Fließen des Urins an der Beutelwandung erfolgt, sondern daß der Urin aus der Röhrchenöffnung frei in den Meßbeutelteil 4 hineintropft, so daß von außen sofort ersichtlich ist, ob zu dem Kontrollzeitpunkt Urin vom Patienten abgegeben wird oder nicht.

Die Herstellung des Behälters erfolgt in an sich bekannter Weise aus entsprechender Kunststoffolie, wobei aber die Erfindung nicht auf den Einsatz dieser Folie beschränkt ist.

Mit dem vorbeschriebenen Auffangbehälter wird mit einem einzigen Beutel ein Gerät geschaffen, das einen Feinstmeßbereich am unteren Ende des Meßbeutelteiles 4 aufweist, einen Feinmeßbereich im oberen Bereich des Meßbeutelteiles 4 und einen Grobmeßbereich, der durch den eigentlichen Hauptbeutelteil 1 gebildet wird. Der Behälter ist allseits geschlossen und ermöglicht somit ein sogenanntes »geschlossenes System«, d. h. es sind Entleerungen des Behälters möglich, ohne daß die Katheterverbindung zwischen Patient und Behälter unterbrochen werden muß, so daß Infektionen nicht mehr möglich sind. Eine Entlüftung wie bei den bisher bekannten Urometern ist nicht erforderlich.

Während bei der Darstellung gemäß den Fig. 3 und 4 der Meßbeutelteil 4 unmittelbar an der Wandung des Hauptbeutelteiles 1 anliegt, ist es durchaus möglich, den Beutel so aufzuhängen, oder einen Steg in den Knickbereich zwischen dem Hauptbeutelteil 1 und dem Meßbeutelteil 4 einzuschalten, daß hier ein gewisser Abstand entsteht, so daß bei Füllen des Meßbeutelteiles 4 die Beutelwandung nicht zur Anlage an den Hauptbeutelteil 1 kommt, so daß dadurch Beeinflussungen des Meßergebnisses nicht möglich sind.

**Patentansprüche**

1. Flexibel ausgebildeter, einteiliger Auffangbehälter für medizinische Zwecke mit einem Meßbeutelteil (4), einem Hauptbeutelteil (1) einer Aufhängeeinrichtung (2, 3), bei dem der mit einem Einlaufröhrchen (5) ausgerüstete, eine Meßskaleneinteilung (7) aufweisende und sich im Querschnitt verjüngende Meßbeutelteil (4) dem Hauptbeutelteil (1) in Strömungsrichtung vergeschaltet ist und am Auslaßende unter Freihalten eines Durchganges einteilig in den Hauptbeutelteil (1) übergeht, dadurch gekennzeichnet, daß der Meßbeutelteil (4) sich zum Einlaßende hin verjüngt und in die Gebrauchslage um eine im Bereich der Aufhängeeinrichtung (2, 3) liegende Knickkante umklappbar ist, daß der Nullwert der Meßskaleneinteilung (7) am äußersten Meßbeutelteilende beginnt und daß das Einlaufröhrchen (5) an seinem freien Ende innerhalb des Meßbeutelteiles (4) abgebogen ist (bei 6).

2. Auffangbehälter nach Anspruch 1, dadurch gekennzeichnet, daß das Ende des Einlaufröhrchens (5) mit einer innerhalb des freien Rohrquerschnitts liegenden Tropfnase (11) ausgerüstet ist.

3. Auffangbehälter nach Anspruch 1 und 2, gekennzeichnet durch ein Entnahmeröhrchen (8), das mit einer Durchstechkappe (9) am Beutelrand mündet und in den Hauptbeutelteil (1) reicht.

4. Auffangbehälter nach Anspruch 1, dadurch gekennzeichnet, daß der Meßbeutelteil (4) sich in der Beutelebene konisch verjüngt.

5. Auffangbehälter nach Anspruch 1, dadurch gekennzeichnet, daß der Meßbeutelteil (4) sich außermittig an den Hauptbeutelteil (1) anschließt.

6. Auffangbehälter nach Anspruch 1 und 2, dadurch gekennzeichnet, daß sich das Ende des Einlaufröhrchens (5) konisch erweitert.

7. Auffangbehälter nach Anspruch 1 und 3, dadurch gekennzeichnet, daß das Entnahmeröhrchen (8) in seinem Endbereich perforiert ist.

8. Auffangbehälter nach einem oder mehreren der vorhergehenden Ansprüche, dadurch gekennzeichnet, daß der Beutelrand mit Ausnahme des Einlaufröhrchens (5) und der Durchstechkappe (9) dicht verschweißt ist.

**Claims**

1. Collecting container for medical purposes, of flexible, one-piece design, with a main bag portion (1), a measuring-bag portion (4), and a suspension arragement (2, 3), in which container the measuring-bag portion (4), which is of tapering cross-section, possesses measuring praduations (7), and is provided with a small-diameter inlet tube (5), is located in advance of the main bag portion (1), in the direction of flow, and at the outlet end merges integrally into the main bag portion (1), while keeping a through-passage open, characterised in that the measuring-bag portion (4) tapers towards the inlet end and, in the use position, can be folded over, about a bend edge which is located in the region of the suspension arrangement (2, 3), in that the zero value of the measuring graductions (7) starts at the outermost end of the measuring-bag portion (4), and in that the small-diameter inlet tube (5) is bent over, at its free end, at (6), inside the measuring-bag portion (4).

2. Collecting container according to claim 1, characterised in that the end of the small-diameter inlet tube (5) is provided with a drip nose (11) which is located inside the bore of the tube (5).

3. Collecting container according to claims 1 and 2, characterised by a small-diameter tapping-off tube (8) which opens at the edge of the bag, with a pierceable cap (9), and which extends into the main bag portion (1).

4. Collecting container according to claim 1, characterised in that the measuring-bag portion (4) tapers conically in the plane of the bag.

5. Collecting container according to claim 1, characterised in that the measuring-bag portion (4) joins the main bag portion (1) asymmetrically.

6. Collecting container according to claims 1 and 2, characterised in that the end of the small-diameter inlet tube (5) widens conically.

7. Collecting container according to claims 1 and 3, characterised in that the small-diameter tapping-off tube (8) is perforated in its end region.

8. Collecting container according to one or more of the preceding claims, characterised in that the edge of the bag is sealed by welding, with the exception of the small-diameter inlet tube (5) and the pierceable cap (9).

**Revendications**

1. récipient collecteur souple d'une seule pièce à usage médical, comportant une partie de sac de mesure (4), une partie de sac principal (1) et un dispositif d'accrochage (2, 3), dans lequel la partie de sac de mesure (4) équipée d'un tube d'entrée (5), présentant une graduation de mesure (7) et rétrécissant en section est placée avant la partie de sac principal (1) dans le sens d'écoulement et rejoint d'une seule pièce la partie de sac principal 6;7 à l'extrémité de sortie en ménageant un passage, caractérisé par le fait que la partie de sac de mesure (4) rétrécit vers l'ouverture d'entrée et peut être rabattue à la position d'utilisation autour d'une arête de pliage située dans la région du dispositif d'accrochage (2, 3), que le zéro de la graduation de mesure (7) commence à l'extrémité extérieure de la partie de sac de mesure et que le tube d'entrée (5) est recourbé à son extrémité libre à l'intérieur de la partie de sac de mesure (4).

2. Récipient collecteur selon la revendication 1, caractérisé par le fait que l'extrémité du tube d'entrée (5) est munie d'un rejeteau situé à l'intérieur de la section libre du tube.

3. Récipient collecteur selon les revendications 1 et 2, caractérisé par un tube de prélèvement (8) qui débouche au bord du sac par un capuchon d'insertion (9) et pénètre dans la partie de sac principal.

4. Récipient collecteur selon la revendication 1, caractérisé par le fait que la partie de sac de mesure (4) rétrécit coniquement dans le plan du sac.

5. Récipient collecteur selon la revendication 1, caractérisé par le fait que la partie de sac de mesure (4) se raccorde de façon excentrée à la partie de sac principal (1).

6. Récipient collecteur selon les revendications 1 et 2, caractérisé par le fait que l'extrémité du tube d'entrée (5) s'élargit coniquement.

7. Récipient collecteur selon les revendications 1 et 3, caractérisé par le fait que le tube de prélèvement (8) est perforé dans sa région terminale.

8. Récipient collecteur selon une ou plusieurs des revendications précédentes, caractérisé par le fait que le bord du sac est soudé de façon étanche, à l'exception du tube d'entrée (5) et du capuchon d'insertion (9).

**Fig.1**

**Fig.2**

**Fig.4**

**Fig.5**

4

5

8

4

10

1

**Fig.5**

11